# EUROPÄISCHE PATENTSCHRIFT

(11) **EP 2 801 263 B1**
(45) Veröffentlichungstag und Bekanntmachung des Hinweises auf die Patenterteilung: **14.04.2021**
(21) Anmeldenummer: 13167190.1
(22) Anmeldetag: 09.05.2013
(51) Int. Cl.: A23L 27/00, A23L 27/10, A23L 27/12, A23G 4/00, A61Q 11/00, A61Q 13/00, C11B 9/00

(54) **Verfahren zur Herstellung einer kühlende Zusammensetzung in Form eines Granulats**
Process for the preparation of a cooling composition in the form of granules
Procédé de préparation d'une composition réfrigérante sous forme de granulés

(43) Veröffentlichungstag der Anmeldung: 12.11.2014
(73) Patentinhaber: Symrise AG, 37603 Holzminden (DE)
(72) Erfinder: Weissbrodt, Jenny, 37603 Holzminden (DE); Asche, Jessica, 37603 Holzminden (DE); Schoppmeier, Thomas, 37603 Holzminden (DE)
(74) Vertreter: Eisenführ Speiser

(56) Entgegenhaltungen:
- EP-A1- 1 011 643
- EP-A1- 2 106 704
- EP-A2- 0 413 539
- EP-A2- 2 033 688
- WO-A1-00/36931
- WO-A1-97/16078
- WO-A1-2011/159935
- DE-A1-102008 000 265
- US-A1- 2003 152 629
- US-A1- 2011 081 303
- AMANDA L. SCHOBER ET AL: "Flavor Release and Perception in Hard Candy: Influence of Flavor Compound-Flavor Solvent Interactions", JOURNAL OF AGRICULTURAL AND FOOD CHEMISTRY, Bd. 52, Nr. 9, 1. Mai 2004 (2004-05-01), Seiten 2628-2631, XP055091250, ISSN: 0021-8561, DOI: 10.1021/jf0354287
- RAJESH V. POTINENI ET AL: "Influence of Flavor Solvent on Flavor Release and Perception in Sugar-Free Chewing Gum", JOURNAL OF AGRICULTURAL AND FOOD CHEMISTRY, Bd. 56, Nr. 9, 1. Mai 2008 (2008-05-01), Seiten 3254-3259, XP055091252, ISSN: 0021-8561, DOI: 10.1021/jf072783e
- Sa-Won Lee ET AL: "Encapsulation of ethanol by spray drying technique: effects of sodium lauryl sulfate", INTERNATIONAL JOURNAL OF PHARMACEUTICS, vol. 187, no. 2, 4 October 1999 (1999-10-04), pages 193-198, XP055611369, NL ISSN: 0378-5173, DOI: 10.1016/S0378-5173(99)00185-4
- Seid Mahdi Jafari ET AL: "Encapsulation Efficiency of Food Flavours and Oils during Spray Drying", DRYING TECHNOLOGY., vol. 26, no. 7, 1 July 2008 (2008-07-01), pages 816-835, XP055269241, US ISSN: 0737-3937, DOI: 10.1080/07373930802135972

## Beschreibung

### Gebiet der Erfindung

Die Erfindung befindet sich auf dem Nahrungsmittelgebiet und betrifft ein neues Verfahren zur Herstellung einer kühlenden Zusammensetzung in Form eines Granulats (wie hierin beschrieben), die in den zu verwendenden Zubereitungen eine physiologisch kühlende Wirkung zur Vermittlung eines Frischegefühls bewirkt.

### Stand der Technik

Kaugummis bestehen vorzugsweise aus einer in Wasser unlöslichen Kaumasse, die selbst bei langem und intensiven Kauen im Mund des Verbrauchers zurückbleibt. Zusätzlich zu dieser Kaumasse enthalten Kaugummis beispielsweise Geschmacksstoffe, Süßungsmittel und Stoffe mit physiologischer Kühlwirkung, die beim Kauen freigesetzt werden und eine erwünschte Wirkung erzielen.

Insbesondere bei der Verwendung von Kühlwirkstoffen wird man vor der Herausforderung gestellt, neben einem möglichst hohen Impact auch eine möglichst langanhaltendende Kühlwirkung zu erzeugen. Allerdings ist es mit bekannten Formulierungen oftmals nicht oder nur sehr eingeschränkt möglich, einen besonders schnellen und hohen Impact oder / und eine besonders langanhaltende Kühlwirkung zu erzeugen. Die Kühlwirkung setzt deshalb oft sehr verzögert ein oder / und lässt sehr schnell nach.

Im Stand der Technik ist bereits bekannt, dass die Geschmackswahrnehmung des Kaugummis während des Kauens verbessert und verlängert werden kann, wenn die Freisetzung von Süßungsmitteln kontrolliert und verlängert wird. Oftmals werden Einkapselungsmittel eingesetzt, um eine frühe und schnelle Freisetzung von Süßungsmitteln zu retardieren, um so die Freisetzung von Süßungsmitteln zu kontrollieren bzw. retardierend freizusetzen. Als Einkapselungsmaterialien kommen dabei solche infrage, die für Lebensmittel geeignet sind, so z.B Shellack von Lebensmittelqualität, das auf unterschiedliche Weise auf das Kaugummi aufgebracht werden kann, wie z.B. durch Nassgranulation, durch Wachsgranulation, durch Sprühtrocknen, durch Sprühkühlen, durch Wirbelschicht-Beschichten, durch Koazervation und dergleichen.

In EP1011643 A1 wird beispielsweise die Herstellung von Kaugummis offenbart. Dort werden acyclische Carboxamide als Kühlwirkstoffe, beispielsweise mit einem Einkapselungsmittel, ausgewählt aus Maltodextrin und Akazie gemischt, um den Kühleffekt zu beeinflussen. Diese Mischung wird dann in eine Kaugummiformulierung gegeben. Auch die internationale Anmeldung WO2011/159935 A1 offenbart Kaugummis mit einer Kombination aus verschiedenen Kühlwirkstoffen.

EP0413539 A2 offenbart ein System zur Abgabe von Aromen, welches eine längere Geschmackserhaltung und eine verbesserte Aromen-Freisetzung bereitstellt, wenn dieses in Kaugummis eingearbeitet wird. Auch die Patentanmeldung US20030152629 A1 offenbart ein System zur Abgabe von Aromen, welches sprühgetrocknete Partikel beinhaltet und insbesondere in oralen Zubereitungen eingesetzt werden kann.

Im Allgemeinen umfassen die Einkapselungsmittel eine Vielzahl von unterschiedlichen Einkapselungsmaterialien von Lebensmittelqualität, die jedoch recht teuer sein können und nicht so wirksam sind, um die Freisetzung von Geschmacksstoffen, Süßungsmitteln und Kühlwirkstoffen in dem gewünschten Ausmaß zu verlängern.

So beschäftigt sich beispielsweise die Patentanmeldung DE 102008000265 A1 mit der Sprühgranulierung von Menthol zur Herstellung von Menthol in Granulatform und Einarbeitung in Verbrauchsgüter, wie zum Beispiel Kaugummis. Auch WO 0036931 offenbart sprühgetrocknete Aromen-Zusammensetzungen, welche in Kaugummis eingesetzt werden können.

Weiterhin offenbart EP 2106704 A1 sprühgetrocknete Partikel mit einem hohen Anteil an Parfüm oder Aromaöl, welche auch in Kaugummis eingearbeitet werden können. In der Anmeldung EP 2033688 A2 werden Oxalsäurederivate offenbart, welche als Kühlwirkstoffe verwendet werden können. Hierin werden auch sprühgetrocknete Granulate enthaltend die beschriebenen Oxalsäurederivate beschrieben.

Es besteht ein Bedarf, die sensorischen Eigenschaften, wie insbesondere die Kühlwirkung bzw. das Frischegefühl bei oralen Zubereitungen, insbesondere bei Kaugummis und Bonbons, während des Verzehrs, aufrechtzuerhalten, zu verlängern, zu verbessern, zu verstärken und den Impact zu verbessern.

Die Aufgabe der vorliegenden Erfindung hat darin bestanden, ein Verfahren zur Herstellung einer Zusammensetzung in Form eines Granulats Verfügung zu stellen, die eine langanhaltende und / oder eine schnell einsetzende kühlende Wirkung in oralen Zubereitungen, insbesondere Kaugummis und Bonbons, entfaltet. Ferner dient die Zusammensetzung der Verstärkung, der Verlängerung, der Verbesserung der Kühlwirkung spezieller Kühlwirkstoffe. Weiterhin sollte diese kühlende Zusammensetzung in orale Zubereitungen, insbesondere in Kaugummis oder anderen Konfekten einzuarbeiten sein. Dabei bestand die Aufgabe der Erfindung besonders darin, ein Verfahren bereitzustellen, um die kühlende Zusammensetzung in solchen oralen Zubereitungen stabil einzuarbeiten, insbesondere ohne dass die Kühlwirkung dabei verloren geht oder zerstört wird.

### Beschreibung der Erfindung

Hierin allgemein beschrieben ist eine Zusammensetzung mit kühlendem Effekt, umfassend etwa 2 bis etwa 15 Gew.-% Kühlwirkstoff (A), 0 bis etwa 35 Gew.-% Alkohol (B), etwa 55 bis etwa 95 Gew.-% hydrophobe Verbindung (C), die in den zu verwendeten oralen Zubereitungen eine physiologisch kühlende Wirkung zur Vermittlung eines Frischegefühls bewirkt.

Hierbei ist es so, dass der Kühlwirkstoff der kühlenden Zusammensetzung auf der der Haut und auf der (Mund-)Schleimhaut eine Empfindung von Kühle aus löst. Dieses Empfinden wird durch eine Interaktion der Kühlwirkstoffe mit Thermorezeptoren, die in der Haut bzw. (Mund-)Schleimhaut sind, erzeugt. Dabei binden die Kühlwirkstoffe an Kälterezeptoren der Haut bzw. (Mund-)Schleimhaut, wodurch die intrazelluläre Calciumkonzentration erhöht wird und einen Nervenreiz auslöst, der im Körper ein Kälteempfinden erzeugt bzw. als Kältegefühl wahrgenommen wird. Ein solcher Rezeptor ist beispielsweise TRPM8, das zu der Gruppe der Kälte-Menthol-Rezeptoren (auch bezeichnet als Cold-Membrane Receptor (CMR)1) bzw. zur Familie der "Transient Receptor Potential Ion Channels" gehört. Dieser Rezeptor wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca2+ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP Channel that senses cold Stimuli and menthol.Cell. 2002 Mar 8; 108(5):705-15; McKemy DD et al. Identification of a cold receptor reveals a general role for TRP Channels in thermosensation Nature 2002 Mar 7; 416 (6876): 52-8; Zuker CS. Neurobiology: A cool ion Channel Nature 2002 Mar 7; 416 (6876): 27-8).

Im Zusammenhang mit der vorliegenden Erfindung wurde überraschenderweise gefunden, dass der Zusatz der oben genannten Zusammensetzung nicht nur einen kühlenden Effekt aufweist, sondern auch durch die Art der Herstellung der Zusammensetzung der Kühleffekt schnell und mit hohem Impact einsetzt, verstärkt wird, verbessert wird und / oder langanhaltend ist. Orale Zubereitungen, die eine solche kühlende Zusammensetzung enthalten, können ebenso die genannten Vorteile der oben genannten Zusammensetzung aufweisen.

Entsprechend den beigefügten Patentansprüchen betrifft der Gegenstand der Erfindung ein Verfahren zur Herstellung einer kühlenden Zusammensetzung, wie in den Ansprüchen 1 bis 3 beschrieben. Dabei wird die kühlende Zusammensetzung in einem Sprühgranulation-prozess verkapselt, wobei der Kühlwirkstoff (A) in einem vorgelagerten Schritt in (B) und (C) gelöst wird, so dass eine homogene Mischung entsteht, und wobei die Temperatur bei 40°C bis 100°C, vorzugsweise bei 45°C bis 80°C, besonders bevorzugt bei 50°C bis 70°C und ganz besonders bevorzugt bei 40°C bis 50°C, zum vollständigen Lösen von (A) gehalten wird. Im weiteren Sprühgranulation- Verarbeitungsprozess wird die Temperatur bei 35°C bis 65°C, bevorzugt bei 38°C bis 60°C, besonders bevorzugt bei 39°C bis 43°C gehalten, um eine Rekristallisation von (A) zu verhindern, wobei die aus der Sprühgranulation erhaltenen Granulatpartikel eine durchschnittliche Teilchengröße von 0.3 mm bis 0.9 mm, bevorzugt von 0.4 mm bis 0.8 mm, besonders bevorzugt von 0.5 mm bis 0.7 mm aufweisen.

Es hat sich als besonders vorteilhaft erwiesen, wenn das Verhältnis von Kühlwirkstoff (A) : Alkohol (B) : hydrophobe Verbindung (C) bei 1:1:8, vorzugsweise bei 1:1.5:7.5, besonders bevorzugt bei 1:3:6 liegt.

Eine Konzentration von 8 bis 10 Gew.-% Kühlwirkstoff (A), 15 bis 35 Gew.% Alkohol (B) und 60 bis 80 Gew.% hydrophobe Verbindung (C), und ganz besonders von 9.5 bis 10 Gew.-% Kühlwirkstoff (A), 25 bis 30 Gew.% Alkohol (B) und 60 bis 75 Gew.% hydrophobe Verbindung (C) ist besonders wirkungsvoll und lässt sich gemäß der oben genannten Herstellungsweise darstellen, ohne dass der Kühleffekt anschließend in oralen Zubereitungen, insbesondere in Kaugummis oder Bonbons verloren geht.

In einer bevorzugten Ausführungsform liegt die Konzentration vom Kühlwirkstoff bei 10 Gew.-%, vom Alkohol (B) bei 30 Gew.% und von der hydrophoben Verbindung (C) bei 60 Gew.%.

Hierin beschrieben ist auch eine orale Zubereitung, umfassend eine kühlende Zusammensetzung mit den Komponenten (A), (B) und (C), wie oben beschrieben, wobei die orale Zubereitung bevorzugt ein Kaugummi oder ein Bonbon ist. Unter dem Begriff Bonbon werden bevorzugt Hartkaramellen und Kaubonbons verstanden.

Weiterhin beschrieben ist eine orale Zubereitung, welche ein Kaugummi ist und a) 5-95 Gew.% Kaugummibasis, b) 5-95 Gew.% Füll- und Süssungsmittel, c) 0.1 -15 Gew.% Geschmackstoffe, d) 0.4-2 Gew.% eine kühlende Zusammensetzung (wie hierin beschrieben), die in den zu verwendeten Zubereitungen eine physiologisch kühlende Wirkung und ein langanhaltendes Frischegefühl bewirkt, umfasst.

Bei der Untersuchung der sensorischen Eigenschaften von oralen Zubereitungen, insbesondere Kaugummis gegenüber konventionellen Kaugummis, also ohne die Kombination aus Kühlwirkstoff (A), Alkohol (B) und hydrophobe Verbindung (C), die gemäß der beschriebenen Sprühgranulationsverfahren hergestellt sind, zeigt sich, keine Kühlwirkung.

### Kühlwirkstoff A

Als Kühlwirkstoff (A) geeignet sind solche Verbindungen, die ausgewählt sind aus der Gruppe, bestehend aus acyclische Carboxamidverbindungen und Mentholverbindungen oder Mischungen daraus. (Die erfindungsgemäß zu verwendenden Kühlwirkstoffe ergeben sich aus den beigefügten Patentansprüchen.)

Die Kombination von mehreren Wirkstoffkomponenten, wie Mischungen aus mehreren Kühlwirkstoffen hat den Vorteil, dass diese sich gegenseitig synergetisch unterstützen.

### Mentholverbindunqen

Mentholverbindungen die im Zusammenhang mit der Erfindung eingesetzt werden können, sind ausgewählt aus der Gruppe, die gebildet wird von Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Alle diese Stoffe sind im Handel erhältlich und nach den üblichen Methoden der organischen Chemie erhältlich.

Ein erster wichtiger Vertreter stellt das Monomenthyl Succinate (FEMA GRAS 3810) dar, das als Stoff bereits 1963 von Brown & Williamson Tobacco Corp. patentiert worden **(**US 3,111,127**)** und als Kühlwirkstoff Gegenstand der Schutzrechte US 5,725,865 und 5,843,466 **(V.Mane Fils)** ist. Sowohl das Succinat als auch das analoge Monomenthyl

Glutarate (FEMA GRAS 4006) stellen wichtige Vertreter von Monomenthylestern auf Basis von Di- und Polycarbonsäuren dar:

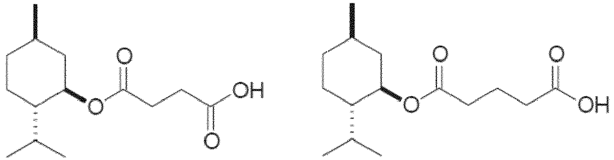

Beispiele für Anwendungen dieser Stoffe finden sich beispielsweise in den Druckschriften WO 2003 043431 (Unilever) oder EP 1332772 A1 (IFF).

Die nächste wichtige Gruppe von im Zusammenhang mit der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten:

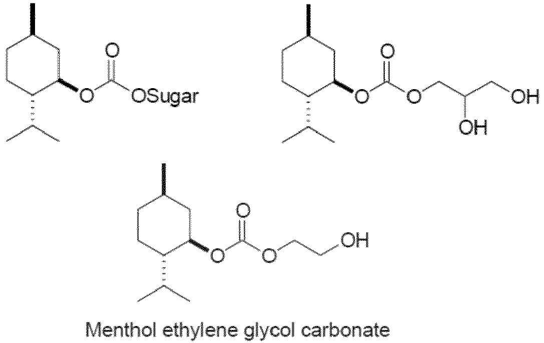

Die Verwendung derartiger Stoffe als Kühlwirkstoff für Zigaretten ist beispielsweise Gegenstand der Druckschrift US 3,419,543 (Mold et al.) aus dem Jahre 1968; die Anwendung als physiologisches Kühlwirkstoff wird in DE 4226043 A1 (H&R) beansprucht.

Im Sinne der Erfindung bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird.

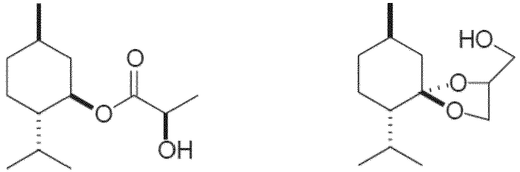

Erstere Struktur wird durch Veresterung von Milchsäure mit Menthol, letztere durch Acetalisierung von Menthon mit Glycerin gewonnen (vgl. DE 2608226 A1**,** H&R). In diese Gruppe von Verbindungen gehört auch das 3-(I-Menthoxy)-1,2,propandiol, das auch als Cooling Agent 10 bekannt ist (FEMA GRAS 3784, vgl. US 6,328,982**,** TIC), sowie das 3-(I-Menthoxy)-2-methyl-1,2,propandiol (FEMA GRAS 3849), das über eine zusätzliche Methylgruppe verfügt.

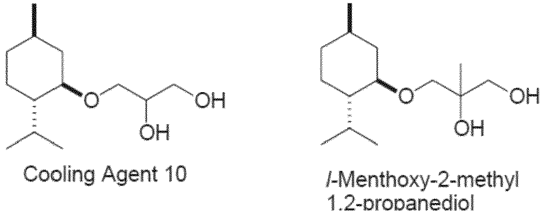

Die Herstellung des 3-(I-Menthoxy)-1,2,propandiol erfolgt beispielsweise ausgehend von Menthol nach dem folgenden Schema (vgl. US 4,459,425**,** Takagaso):

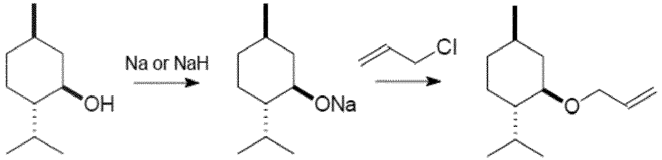

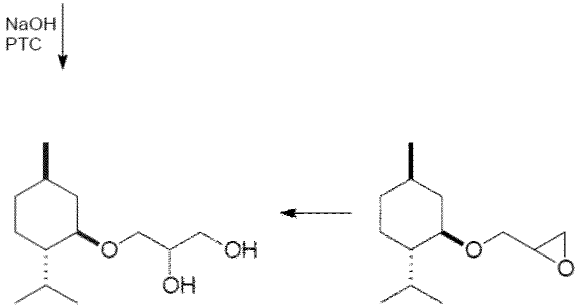

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

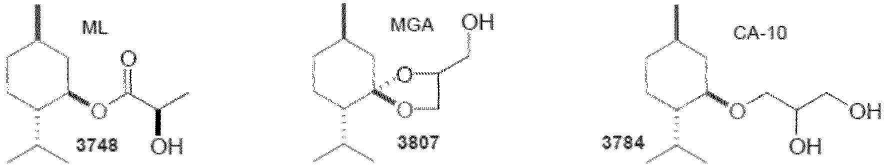

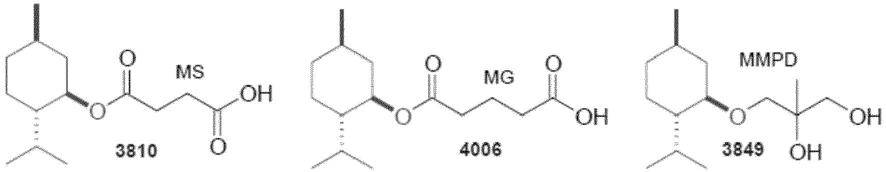

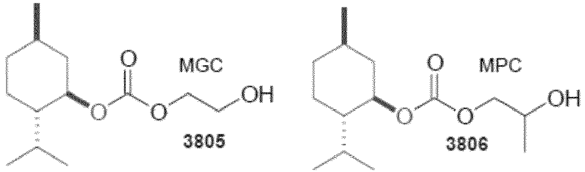

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonat erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14, WS 23 und WS-30. Die beiden nachfolgenden Schaubilder zeigen die Synthesewege auf:

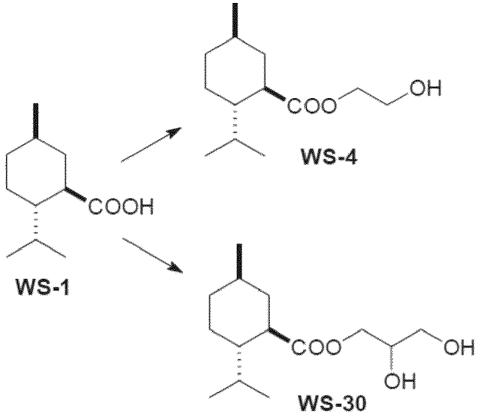

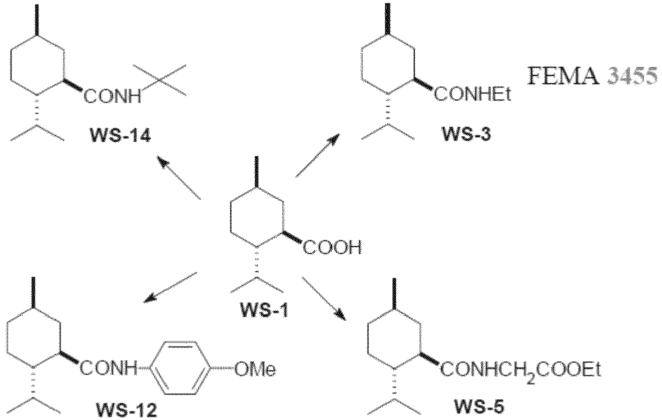

Die sich von WS-1 ableitenden Ester werden beispielsweise in US 4,157,384**,** die entsprechenden N-substituierten Amide in J. Soc. Cosmet. Chem. S. 185-200 (1978**)** beschrieben.

### Acyclische Carboxamidverbindunqen

Bevorzugte acyclische Carboxamide leiten sich von der folgenden Struktur ab: 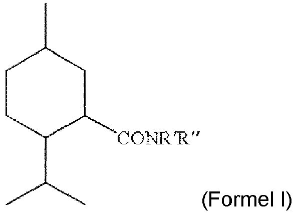 wobei R' und R" unabhängig voneinander Wasserstoff, Hydroxylgruppe oder ein Alkylrest mit bis zu 25 C-Atomen, eine Arylgruppe mit bis zu 10 C-Atomen, ausgewählt aus substituierten und unsubstituierten Phenyl-, Phenylalkyl-, Naphthyl- und Pyridylresten sein kann. Der Alkylrest kann dabei sowohl verzweigt, unverzweigt als auch cyclisch sein, so dass Alkyl-, Cycloalkyl-, Alkenyl-, Cycloalkenyl-, Alkynyl-, Hydroxyalkyl-, Acyloxyalkyl-, Alkoxy-, Alkoxyalkyl-, Aminoalkyl-, Acylaminoalkyl-, Carboxyalkylreste und ähnliche Kombinationen umfasst sind.

Bevorzugt sind R' und R" Methyl, Ethyl, Propyl, Butyl, Isobutyl, n-Decyl, Cyclopropyl, Cyclohexyl, Cyclopentyl, Cycloheptylmethyl, 2-Hydroxyethyl, 3-Hydroxy-n-propyl, 6-Hydroxy-n-hexyl, 2-Aminoethyl, 2-Acetoxyethyl, 2-Ethylcarboxyethyl, 4-Hydroxybut-2-ynyl, Carboxymethyl, Benzyl, Naphthyl, 4-Methoxyphenyl, 4-Hydroxyphenyl, 4-Methylphenyl, 3-Hydroxy-4-methylphenyl, 4-Fluorophenyl, 4-Nitrophenyl, 2-Hydroxynaphthyl, Pyridyl.

Ganz besonders bevorzugt ist der Kühlwirkstoff A (*1R*,*2S*,*5R*)-N-(4-methoxyphenyl)-5-methyl-2-(1-methylethyl)cyclohexane-carboxamide (FEMA 4681), das die folgende Struktur (II) aufweist:

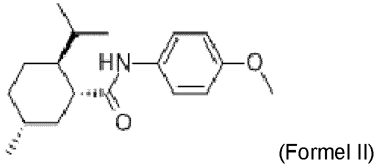

### Alkohol B

Als Alkohol (B) geeignet sind solche Verbindungen, die ausgewählt sind aus der Gruppe, bestehend aus C1 bis C3 Alkohole oder Mischungen daraus, wie Methanol, Ethanol, Propanol, 1,2-Propandiol. Besonders bevorzugt ist Ethanol und 1,2-Propandiol.

### Hydrophobe Verbindung C

Geeignete hydrophobe Verbindungen (C) sind ausgewählt aus der Gruppe, bestehend aus ätherischen Ölen (natürliche Öle), Neutralöl und Pflanzenölen (pflanzliche Öle) und Mischungen daraus.

Bevorzugte ätherische Öle sind dabei ausgewählt aus der Gruppe, bestehend aus wie Pfefferminzöl (Menthol), Carvon, Eukalyptusöl, Grapefruit, Orangenöl, Zitronenöl, Terpentinöl, Teebaum- und Nelkenöl, Kampfer, Rosenöl, Lavendelöl oder Methylsalicylat oder Mischungen daraus.

Bevorzugte Pflanzenöle sind aus Ölsaaten gewonnene Fette und Öle und sind ausgewählt aus der Gruppe, bestehend aus Sonnenblumen-, Oliven, -Distelöl, Sesam- und Mandelöl, Algenöl, Aprikosenkernöl oder Marillenkernöl, Arganöl, Avocadoöl, Borretschöl oder Borretschsamenöl, Cashew-Schalenöl, Hagebuttenkernöl, Haselnussöl, Jojobaöl, Kaffeebohnenöl, Kamelienöl, Macadamiaöl, Mandelöl, Papayasamenöl, Pistazienöl, Rizinusöl, Sanddornöl, Sanddornkernöl, Walnussöl oder Mischungen daraus.

Bevorzugte Neutralöle (MCT-Öl= engl.: *medium chain triglycerides,)* sind Mischungen aus mittelkettigen Fettsäuren (Triglyceriden), genauer gesagt eine Mischung aus Caprinsäure und Caprylsäure (CAS-Nummer 73398-61-5).

Ausgangsbasis für die Herstellung der Neutralöle sind Palmkern- oder Kokosöl. Die Fette werden verseift (durch Hydrolyse gespalten), unerwünschte Fettsäuren (u. a. Laurinsäure, Myristinsäure) abgetrennt und die gewünschten Fettsäuren wieder mit Glycerin verestert. MCT-Triglyceride sind beispielsweise kommerziell erhältlich unter den Handelsnamen *Mygliol 812*® der Firma Sasol, *Myritol 312*® der Firma Cognis und *Tegosoft*® *CT* der Firma Evonik. Die verschiedenen Produkte unterscheiden sich in der prozentualen Komposition der beiden Fettsäuren, Caprinsäure und Caprylsäure. Alle sind Lipide auf Basis von ca. 50-65 % Caprylsäure (C8:0) und ca. 30-45 % Caprinäure (C10:0); Capronsäure (C6:0), Laurinsäure (C12:0) und Myristinsäure (C14:0) sind in sehr geringen Anteilen vorhanden.

### Herstellverfahren

Die kühlende Zusammensetzung wird in einem Sprühgranulation-prozess hergestellt, wobei die Komponenten Kühlwirkstoff (A), Alkohol (B) und hydrophobe Verbindung (C) verkapselt werden. Wichtig ist hierbei, dass der Kühlwirkstoff (A) in einem vorgelagerten Schritt in (B) und (C) vollständig gelöst wird, so dass eine homogene Mischung entsteht, wobei die Temperatur bei 40°C bis 100°C, zum vollständigen Lösen von (A) gehalten wird. Alle Komponenten werden anschließend in einem Sprühgranulation-Verarbeitungsprozess verkapselt. Dabei wird die Temperatur dann bei 35°C bis 65°C, bevorzugt bei 38°C bis 60°C, besonders bevorzugt bei 39°C bis 43°C gehalten, um eine Rekristallisation des Kühlwirkstoffes (A) zu verhindern. Die aus der Sprühgranulation erhaltenen Granulatpartikel weisen eine durchschnittliche Teilchengröße von 0.3 mm bis 0.9 mm, bevorzugt von 0.4 mm bis 0.8 mm, besonders bevorzugt bei 0.5 mm bis 0.6mm auf. Bei der Herstellung der kühlenden Zusammensetzung ist es besonders wichtig darauf zu achten, dass der Kühlwirkstoff (A) vollständig gelöst wird. Dieses wird zum einen erreicht durch die Temperatur, und zum anderen durch die spezielle Kombination von (A), (B) und (C). Das Lösen von (A) ist bei 40°C bis 100°C, vorzugsweise bei 45°C bis 80°C, besonders bevorzugt bei 50°C bis 70°C und ganz besonders bevorzugt bei 40°C bis 50°C.

Ebenfalls notwendig ist, dass im anschließenden Sprühgranulationsverfahren die Temperatur so gehalten wird, dass eine Rekristallisation des Kühlwirkstoff (A) verhindert wird, d.h. die Temperatur bei 35°C bis 65°C, bevorzugt bei 38°C bis 60°C, besonders bevorzugt bei 40°C bis 43°C gehalten wird.

In einer bevorzugten Ausführungsform ist der Kühlwirkstoff (A) (*1R,2S,5R*)-N-(4-methoxyphenyl)-5-methyl-2-(1-methylethyl)cyclohexane-carboxamide (FEMA 4681), der Alkohol (B) Ethanol und die hydrophobe Verbindung (C) Neutralöl.

In einer bevorzugten Ausführungsform ist der Kühlwirkstoff (A) (*1R,2S,5R*)-N-(4-methoxyphenyl)-5-methyl-2-(1-methylethyl)cyclohexane-carboxamide (FEMA 4681), der Alkohol (B) 1,2-Propandiol und die hydrophobe Verbindung (C) Neutralöl.

In einer bevorzugten Ausführungsform ist der Kühlwirkstoff (A) (*1R,2S,5R*)-N-(4-methoxyphenyl)-5-methyl-2-(1-methylethyl)cyclohexane-carboxamide (FEMA 4681), der Alkohol (B) Ethanol und die hydrophobe Verbindung (C) Pfefferminzöl.

In diesen Kombinationen lässt sich die kühlende Zusammensetzung besonders gut in ein Kaugummi oder einen Bonbon einarbeiten und es zeigen sich besonders deutlich die weiter oben beschriebenen Vorteile der Erfindung. Insbesondere ermöglichen diese speziellen Kombinationen, dass überhaupt ein physiologischer Kühleffekt in Kaugummis oder Bonbons beim Verzehr wahrgenommen werden kann.

Durch die Wirbelschicht-Sprühgranulation können Wirkstoffe in kompakte und nahezu runde Granulate mit hervorragenden physikalischen Eigenschaften eingekapselt werden. Der Prozess ist im Stand der Technik gut bekannt und wird z.B. in EP1139791 ausführlich beschrieben. In der Wirbelschicht-Sprühgranulation werden feststoffhaltige Flüssigkeiten (z.B. Suspensionen oder Emulsionen) in der Wirbelschicht zerstäubt und treffen in Tropfenform auf die Granulationskeime. Dabei verdunstet die Flüssigkeit und der Feststoff wird auf den Granulationskeim aufgezogen und bildet einen festen Mantel. Dies wiederholt sich im Wirbelbett laufend, so dass sehr kompakte, schalenartig aufgebaute Granulate entstehen. Parameter wie Korngröße, Restfeuchte und Feststoffgehalt lassen sich bei der Wirbelschicht-Sprühgranulation sehr genau einstellen, so dass die unterschiedlichsten Substanzen zu Granulaten verarbeitet werden können. Da bei der Granulation im Wirbelschichtverfahren die Prozesse Trocknung und Formgebung parallel ablaufen, spricht man auch von der kontinuierlichen Wirbelschicht-Sprühtrocknungsgranulation (M.J.V. Goldschmidt, G.G.C. Weijersa, R. Boerefijn, J.A.M. Kuipers: "Discrete element modelling of fluidised bed spray granulation", Powder Technology 138 (2003) 39-45; Maksym Dosta, Stefan Heinrich, Joachim Werther: Fluidized bed spray granulation: "Analysis of the system behaviour by means of dynamic flowsheet simulation", Powder Technology 204 (2010) 71-82).

Beschrieben hierin ist ebenfalls ein Granulat, enthaltend Kühlwirkstoff (A), Alkohol (B) und hydrophobe Verbindung (C), erhältlich nach dem oben beschriebenen Sprühgranulationsverfahren.

Beispielhaft ist dieses Granulat wie folgt zusammengesetzt:

| | **Zusammensetzung** | **D** | **E** |
|---|---|---|---|
| Gemisch A | Modifizierte Stärke | 45-60 | 45-60 |
| | Gummi arabicum (Senegal) | 15-25 | 15-25 |
| | Zuckeralkohol | 5-10 | 5-10 |
| | Farbstoff | 0-2 | 0-2 |
| | Polysaccharide | 0-2 | 0-2 |
| | Geliermittel* | 0-2 | 0-2 |
| | Maltodextrin | - | 50-75 |
| Gemisch B | kühlende Zusammensetzung | 10-30 | 10-30 |

Weiterhin beispielhaft enthält dieses Granulat die Zusammensetzung:

| | **Zusammensetzung** | **D** | **E** |
|---|---|---|---|
| Gemisch A | Modifizierte Stärke | 50,0 | 50,0 |
| | Gummi arabicum (Senegal) | 20,0 | 20,0 |
| | Zuckeralkohol | 7,0 | 7,0 |
| | Farbstoff | - | 0,15 |
| | Polysaccharide | - | 1,5 |
| | Geliermittel* | - | 1,0 |
| | Maltodextrin | - | 68,0 |
| Gemisch B | kühlende Zusammensetzung | 20,0 | 20,0 |

### Gewerbliche Anwendbarkeit

### Kaugummis

Bei der bevorzugten oralen Zubereitung, in der die kühlende Zusammensetzung eingearbeitet wird, handelt es sich um Kaugummis.

Der hier verwendete Ausdruck "Kaugummi" umfasst insbesondere Kaugummidragees, Kaugummistreifen, Kaugummikomprimat, Kaugummikissen, Kaugummiwürfel und auch Bubble-Gums und dergleichen.

Die bevorzugte orale Zubereitung in der die kühlende Zusammensetzung eingearbeitet wird ist ein Kaugummi. Eins solche Zubereitung umfasst vorzugsweise a) 5-95 Gew.% Kaugummibasis, b) 5-95 Gew.% Füll- und Süßungsmittel, c) 0.1 -15 Gew.% Geschmackstoffe, d) 0.4-2 Gew.% kühlende Zusammensetzung. Die kühlende Zusammensetzung bewirkt in den zu verwendeten oralen Zubereitungen, insbesondere im Kaugummi oder Bonbons eine physiologisch kühlende Wirkung, so dass der Verbraucher den Eindruck eines langanhaltenden Frischegefühls vermittelt bekommt.

### Wasserunlösliche Basis

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

### Wasserlösliche Bestandteile

Zusätzlich zu der wasserunlöslichen Gummibasis enthalten Kaugummizubereitungen regelmäßig einen wasserlöslichen Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Rebaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine (Fibersol).

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, Aromen und dergleichen dar, wie etwa Minzöl, Spearmintöl, Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

Ebenfalls hierin beschrieben ist ein Verfahren zur Herstellung eines Kaugummis mit einem länger anhaltenden Frischegefühl, umfassend:
i) Herstellung einer Kaugummizusammensetzung mit 5-95 Gew.% Kaugummibasis, 5-95 Gew.% Füll- und Süßungsmittel, 0.1 -15 Gew.% Geschmackstoffe und
ii) Zugabe von 0.2-3 Gew.% kühlende Zusammensetzung (wie hierin beschrieben), die in den zu verwendeten Zubereitungen eine physiologisch kühlende Wirkung bewirkt zur Vermittlung eines Frischegefühls.

Besonders wirkungsvoll ist eine Konzentration von 0.4-1 Gew.% der kühlenden Zusammensetzung.

### Bonbons

Bei einer weiteren bevorzugten oralen Zubereitung, in der die kühlende Zusammensetzung eingearbeitet wird, handelt es sich um Bonbons.

Bonbons umfassen als Bonbonbasis zumeist Zuckerverbindungen, wie Mono-, Di- und Trisaccharide, Oligosaccharide oder deren Derivate, wie z.B. Glukose, Laktose, Maltose, Xylose, Sucrose und Frucht-oligosacchariden. Dabei sind in einer Bonbonbasis 10 bis 99 Gew.%, vorzugsweise 15 bis 95 Gew.% , besonders bevorzugt 15 bis 95 Gew.% Zuckerverbindungen enthalten.

Weiterhin sind in Bonbons essbare Säuren enthalten, wie aliphatische, gesättigte und ungesättigte Mono-, Di- und Tricarbonsäuren, wie Zitronensäure, Fumarsäure, Maleinsäure, Milchsäure, Weinsäure, Adipinsäure, Bernsteinsäure, Ascorbinsäure, Glutarsäure, Essigsäure, Phoshporsäure usw.

Bonbons (auch Drops) gibt es in fast allen erdenklichen Farben und Geschmacksrichtungen, und Formen. Zumeist haben Bonbons eine ellipsoide Form oder sind Kugelförmig. In der Regel haben Bonbons eine besonders süße, saure oder flüssige Füllung. Man unterscheidet zwischen Hartbonbons, welche ein glasartiges Gefüge aufweisen und einen Wassergehalt von bis zu 3 % haben, und Weichbonbons (Toffee, Toffie), die zäh sind, eine kaugummiartige Konsistenz haben und bis zu 8 % Wasser enthalten.

Bonbons wie hierin beschrieben weisen bevorzugt eine Füllung auf, umfassend der kühlenden Zusammensetzung. Diese Bonbons haben bevorzugt eine Bonbonbasis von 60-95 Gew.%, besonders bevorzugt von 75 bis 85 Gew.% und eine Füllung von 5 bis 40 Gew.%, bevorzugt 15 bis 25 Gew.%. Dabei sind neben der kühlenden Zusammensetzung noch weitere Inhaltstoffe in der Füllung vertreten. Die kühlende Zusammensetzung ist in einer solchen Bonbon in eine Menge von 0.2 bis 4 Gew.%, bevorzugt 0.5 bis 2 Gew.% enthalten.

Die Füllung eines solchen Bonbons kann fest, pulverförmig, wässrig, pastös, gelförmig oder gummiartig sein. Zumeist weist eine wässrige Füllung einen Wassergehalt von weniger als 10%, bevorzugt weniger als 8%, besonders bevorzugt weniger als 6% auf.

Die Füllung kann weiterhin essbare Inhaltstoffe enthalten, die für den Lebensmittelbereich zugelassen sind, so wie Alkohole und Polyalkohole, wie Glyzerin, Polyethylenglykol bzw. Propylenglykole mit geringen Molekulargewichten (weniger 1000 MW). Diese sind im Bereich von 30-95 Gew.%, bevorzugt 40 bis 90 Gew.%, besonders bevorzugt 40 bis 60 Gew.% in der Füllung enthalten.

Ferner können Bonbons Füllungen Süßstoffe in eine Menge von 5 bis 80 Gew.%, vorzugsweise 30 bis 75 Gew.% enthalten. Die eingesetzten Süßstoffe in Bonbons können aus dem Abschnitt für Kaugummi entnommen werden, da sie für Bonbons analog verwendbar sind.

Bonbonsfüllungen umfassen auch Verdickungsmittel, die für den Nahrungsmittelbereich zugelassen sind. Diese sind beispielsweise Xanthan Gummi, Carrageenan und deren Derivate, Hydroxylpropylmethylcellulose, Sklerotium Gummi, Pullulan, Rhamsan Gummi, Welan Gummi, Konjak, Kurdlan, Carbomer, Algin, Alginsäure, Alginate und deren Derivate, Hydroxyethylcellulose und deren Derivate, Hydroxypropylcellulose und deren Derivate, Phosphatstärke-Derivate, Guar Gummi und deren Derivate, Stärke und deren Derivate, Co-polymere von Maleinsäureanhydride mit Alkenen und deren Derivate, Ethylenglycole/Propylenglycol Co-polymere, langkettige Alkohole, wie Behenylalkohol, Poloxamere und deren Derivate, Polyacyralet und deren Derivate, Methylcellulose und deren Derivate, Ethylcellulose und deren Derivate, Agar und deren Derivate, Pektin und deren Derivate, Chitosan und deren Derivate, und höhermolekulare Polyethylenglykole wie Polyethylenglycole (mit 10000 MW oder mehr), Karaya Gummi, Co-polymere von Vinylpyrrolidone mit Alkenen, Polyacrylamide, Chitinderivate, Gelatine, Beta-Glucan, Dextrin, Dextran, Cyclodextrin, Methacrylate, Mikrokristalline Cellulose, Polyquats, Furcellaren gum, Ghatti gum, Psyllium gum, Quince gum, Tamarind gum, Larch gum, Tara gum, Talg, Kaolinischer Ton, Bentonite, Cellulose, Fumed Silica und Mischungen daraus.

Verdickungsmittel sind in Bonbons vorzugsweise in eine Menge von 0.001 bis 10 Gew.%, besonders bevorzugt 0.01 bis 5 Gew.%, ganz besonders bevorzugt 0.01 bis 2.5 Gew.% und insbesondere bevorzugt 0.01 bis 1 Gew.% enthalten.

Aromastoffe können wässrig oder ölbasiert sein und beispielsweise aus Pflanzenteilen, wie Blättern, Blüten, Früchten usw. gewonnen sein. Geeignete Aromastoffe stammen beispielsweise aus den Ölen von Zitronen, Orangen, Bananen, Trauben, Limette, Aprikose, Grapefruit oder aus der Frucht von Apfel, Erdbeere, Kirschen, Orangen, Ananas oder Aromen von Kaffee, Kakao, Cola, Erdnuss, Mandel, Süßholzwurzel oder Ingwerwurzel. Aromastoffe werden in Bonbons in eine Menge vorzugsweise bis zu 4 Gew.%, besonders bevorzugt von 0.1 bis 1 Gew.% eingesetzt.

### Verwendung der Zubereitungen

Schließlich ist hierin die Verwendung der kühlenden Zusammensetzung, um in oralen Zubereitungen ein Frischegefühl durch eine physiologisch kühlende Wirkung zu vermitteln, beschrieben. Die orale Zubereitung ist bevorzugt ein Kaugummi oder ein Bonbon.

### Beispiele

Alle Prozentangaben sind, wenn nichts anderes angegeben ist, auf das Gewicht bezogen.

### I) Herstellung von Granulatpartikeln mit einer hierin beschriebenen kühlenden Zusammensetzung

Ein Gemisch B wird vorgelegt und auf eine Temperatur von 50 °C erwärmt und gerührt bis eine homogene Mischung entsteht. Danach wird eine Trägerkomponente aus einem homogenen Gemisch A bereitgestellt und ebenfalls auf 50°C erwärmt. Anschließend wird das Gemisch B in die Trägerkomponente-Mischung A dispergiert und das gesamte Gemisch sprühgranuliert, wobei die Temperatur während des Prozess bei 39°C bis 40°C gehalten wird.

Die aus der Sprühgranulation erhaltenen Granulatpartikeln können eine durchschnittliche Teilchengröße von 0.3 mm, 0.4 mm, 0.5 mm, 0.6 mm, 0.7 mm, 0.8 mm und 0.9 mm auf weisen, je nach Verfahrensbedingungen.

Die Granulatpartikel aus der Herstellung weisen die in Tabelle 1 aufgeführte Zusammensetzung auf.

**Tabelle 1**

| Granulat enthaltend kühlende Zusammensetzung | | | |
|---|---|---|---|
| | **Zusammensetzung** | **D** | **E** |
| Gemisch A | Modifizierte Stärke | 45-60 | 45-60 |
| | Gummi arabicum (Senegal) | 15-25 | 15-25 |
| | Zuckeralkohol | 5-10 | 5-10 |
| | Farbstoff | 0-2 | 0-2 |
| | Polysaccharide | 0-2 | 0-2 |
| | Geliermittel* | 0-2 | 0-2 |
| | Maltodextrin | - | 50-75 |
| Gemisch B | kühlende Zusammensetzung | 10-30 | 10-30 |

| | | | |
|---|---|---|---|
| *Pektin | | | |

Kühlende Zusammensetzungen können wie folgt zusammengesetzt sein:

**Tabelle 2**

| Kühlende Zusammensetzung | | | | | | | | |
|---|---|---|---|---|---|---|---|---|
| **Zusammensetzung** | **F1** | **F2** | **F3** | **F4ⁱ)** | **F5** | **F6** | **F7** | **F8** |
| Kühlwirkstoff (A)* | 10,0 | 10,0 | 10,0 | 10,0 | 10,0 | 15,0 | 10,0 | 10,0 |
| Alkohol (B)** | - | 10,0 | 15,0 | 30,0 | 90,0 | 85,0 | 20,0 | 15,0 |
| Hydrophobe Verbindung (C)*** | 90,0 | 80,0 | 75,0 | 60,0 | - | - | 70,0 | 75,0 |

| | | | | | | | | |
|---|---|---|---|---|---|---|---|---|
| *Kühlwirkstoff A ausgewählt aus: Menthone Glyceryl Acetal (FEMA GRASⁱ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthoxy-1,2-propanediol (FEMA GRAS 3784), Menthoxy-2-methyl-1,2-propandiol (FEMA GRAS 3849) oder (*1R,2S,5R*)-N-(4-methoxyphenyl)-5-methyl-2-(1-methylethyl) cyclohexane-carboxamide (FEMA 4681). **Alkohol B: Ethanol oder 1,2-Propandiol ***Hydrophobe Verbindungen ausgewählt aus: Pfefferminzöl (Menthol), Carvon oder Neutralöle | | | | | | | | |

### i) Kühlwirkstoff A: (1R,2S,5R)-N-(4-methoxyphenyl)-5-methyl-2-(1-methylethyl) cyclohexane-carboxamide (FEMA 4681), Alkohol B: Ethanol, Hydrophobe Verbindung C: Pfefferminzöl

### II) Kaugummimasse

Aus einem Granulat gemäß Tabelle 1 und einer Kaugummimasse, wie unten in Tabelle 3 angegeben, wurden Kaugummis hergestellt und deren Kühlwirkung sensorisch bewertet.

**Tabelle 3**

| Kaugummimassen | | | |
|---|---|---|---|
| **Zusammensetzung** | **K** | **L** | **M** |
| Polyisobutylen (MW 20.000) | 20.0 | 25.0 | 30.0 |
| Sorbitol | 51,0 | 47,5 | 44,5 |
| Mannitol | 5,0 | 4,3 | 3,6 |
| Glycerin | 8,0 | 8,0 | 7,0 |
| Lycasin:Glycerin (1:1) | 8,2 | 8,0 | 7,0 |
| Lecithin | 0,2 | 0,2 | 0,2 |
| Aromamischung | 1,0 | 1,0 | 1,0 |
| Wasser | Ad 100 | | |

### III) Sensorischer Test

Die erfindungsgemäß hergestellten kühlenden Zusammensetzungen wurden in Kaugummimassen eingearbeitet. Anschließend wurde die Kühlwirkung der Kaugummis von geschulten Personen, sensorisch bewertet. Die sensorische Bewertung ist in Tabelle 4 zusammengefasst, wobei folgende Skalierung verwendet wurde:
Bewertungsbereich 0: nichts wahrnehmbar
Bewertungsbereich 1-3: schwache Kühlwirkung
Bewertungsbereich 4-6: mittlere Kühlwirkung
Bewertungsbereich 7-8: starke Kühlwirkung
Bewertungsbereich 9-10: starke Kühlwirkung

Beispiele E1 bis E4 dienen der Verdeutlichung der Erfindung, Beispiel V1 dem Vergleich.

**Tabelle 4**

| Sensorische Bewertungen von Kaugummis mit und ohne kühlende Zusammensetzung | | | | | |
|---|---|---|---|---|---|
| **Zeit [min]** | **V1** | **E1** | **E2** | **E3** | **E4** |
| 1 | 1-3 | 1-3 | 4-6 | 4-6 | 4-6 |
| 2 | 4-6 | 4-6 | 7-8 | | |
| 3 | | | | | |
| 4 | 7-8 | | 9-10 | | 9-10 |
| 5 | | | | 7-8 | |
| 6 | 9-10 | | | | 7-8 |
| 7 | | | | | |
| 8 | | | | | |
| 9 | | | | | |
| 10 | | | | 4-6 | |
| 15 | | | 4-6 | | 4-6 |
| 20 | | | | | |
| 25 | 4-6 | 1-3 | | | |
| 30 | | | | 1-3 | 1-3 |
| 35 | | | | | |
| 40 | | | | | |
| 45 | | | 1-3 | | |

Es wird an V1 kurz erläutert, wie die Tabelle zu verstehen Ist: In der ersten Minute ist eine schwache Kühlwirkung wahrnehmbar, die in der Zeitspanne von der zweiten bis zu dritten Minute zunimmt in den Bereich mittlere Kühlwirkung geht. In der vierten Minute bis zu 5. Minute zeigt das Produkt eine starke Kühlwirkung, die in der sechsten Minute nochmals steigert zu eine organoleptischen sehr starken Kühlwirkung, die in der 25. Minuten stark abnimmt zu einem mittleren Kühlwirkungseffekt.

Die Kontroll-Formulierung V1 ist eine Standardmischung mit Citrus Mint und wurde als Benchmark verwendet.
E1: 0.4% Granulat D in einer Kaugummimasse gemäß Tabelle 3
E2: 0.6% Granulat D in einer Kaugummimasse gemäß Tabelle 3
E3: 0.4% Granulat E in einer Kaugummimasse gemäß Tabelle 3
E4: 0.6% Granulat E in einer Kaugummimasse gemäß Tabelle 3

Wird der Kühlwirkstoff (A) lediglich in Zuckeralkohol und nicht wie erfindungsgemäß durch Sprühgranulation eingearbeitet, sondern lediglich darin eingeschmolzen und anschließend in eine Kaugummimasse einformuliert, so zeigt sich kein Kühleffekt, wie Tabelle 5 zeigt:

**Tabelle 5**

| Kaugummimasse mit kühlende Zusammensetzung eingearbeitet ohne Sprühgranulation | | | |
|---|---|---|---|
| **Vergleichsversuch** | **V1** | **V2** | **V3** |
| Kühlwirkstoff in Isomalt | 1% | 10% | 10% |
| Isomalt mit Kühlwirkstoff in Kaugummi | 0.4% | 0.2% | 0.8% |
| Zusatz von Dummy* | 1.2% | 1.2% | 1.2 |
| Sensorischer Test | Keine Kühlung | | |

| | | | |
|---|---|---|---|
| *1 Teil Triacetin und 2 Teile MCT-Pflanzenöl | | | |

In der folgenden Tabelle 6 ist eine Beispielformulierung für ein Bonbon:

**Tabelle 6**

| Bonbon enthalten kühlende Zusammensetzung | |
|---|---|
| **Zusammensetzung** | **A** |
| Sucrose | 59.8 |
| Glucose (liq.) | 43.7 |
| Wasser | 16.2 |
| Zitronensäureanhydrid | 1.2 |
| kühlende Zusammensetzung | 0.3 |
| Aroma | 0.2 |

## Patentansprüche

1. Verfahren zur Herstellung einer kühlenden Zusammensetzung in Form eines Granulats, **dadurch gekennzeichnet,**
**dass** eine kühlende Zusammensetzung in einem Sprühgranulation-Prozess verkapselt wird, wobei die kühlende Zusammensetzung eine Zusammensetzung ist, die aus
(A) 8 bis 10 Gew.-% Kühlwirkstoff ausgewählt aus der Gruppe, bestehend aus Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14, WS-23 und WS-30, oder acyclische Carboxamide der Formel (I) 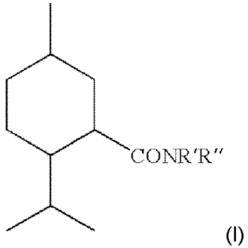 wobei
R' und R" unabhängig voneinander Wasserstoff, oder eine Hydroxylgruppe oder ein verzweigter, unverzweigter, cyclischer Alkylrest mit bis zu 25 C-Atomen, eine Arylgruppe mit bis zu 10 C-Atomen, ausgewählt aus substituierten und unsubstituierten Phenyl-, Phenylalkyl-, Naphthyl- und Pyridylresten sein kann, oder Mischungen daraus, und
(B) mindestens 15 Gew.-% Alkohol ausgewählt aus der Gruppe, bestehend aus einem C1 bis C3-Alkohol oder Mischungen daraus, und
(C) mindestens 60 Gew.-% hydrophobe Verbindung ausgewählt aus der Gruppe, bestehend aus ätherischen Ölen, Neutralölen, Pflanzenölen oder Mischungen daraus,
besteht,
wobei der Kühlwirkstoff (A) in einem vorgelagerten Schritt in (B) und (C) vollständig gelöst wird, so dass eine homogene Mischung entsteht, wobei die Temperatur bei 40°C bis 100°C zum vollständigen Lösen von (A) gehalten wird,
und im weiteren Sprühgranulation-Verarbeitungsprozess die Temperatur bei 35 °C bis 65 °C gehalten wird, um eine Rekristallisation von (A) zu verhindern,
wobei die aus der Sprühgranulation erhaltenen Granulatpartikel der kühlenden Zusammensetzung in Form eines Granulats eine durchschnittliche Teilchengröße von 0,3 mm bis 0,9 mm aufweisen.

2. Verfahren gemäß Anspruch 1, **dadurch gekennzeichnet, dass** der Kühlwirkstoff (A) (1R,25,5R)-N-(4-methoxyphenyl)-5-methyl-2-(1-methylethyl)- cyclohexane-carboxamide (FEMA 4681) ist.

3. Verfahren gemäß einem der Ansprüche 1 oder 2, **dadurch gekennzeichnet, dass** die hydrophobe Verbindung (C) ausgewählt ist aus der Gruppe, bestehend aus Pfefferminzöl (Menthol), Carvon, Eukalyptusöl, Grapefruit, Orangenöl, Zitronenöl, Terpentinöl, Teebaum- und Nelkenöl, Kampfer, Rosenöl, Lavendelöl oder Methylsalicylat, Sonnenblumen-, Oliven, Distelöl, Sesam- und Mandelöl, Algenöl, Aprikosenkernöl oder Marillenkernöl, Arganöl, Avocadoöl, Borretschöl oder Borretschsamenöl, Cashew-Schalenöl, Hagebuttenkernöl, Haselnussöl, Jojobaöl, Kaffeebohnenöl, Kamelienöl, Macadamiaöl, Mandelöl, Papayasamenöl, Pistazienöl, Rizinusöl, Sanddornöl, Sanddornkernöl, Walnussöl oder Neutralöle mit einen Anteil von 50-65 % Caprylsäure, 30-45 % Caprinsäure und einen geringen Anteil von Capronsäure, Laurinsäure und Myristinsäure oder Mischungen daraus.

## Claims

1. Method for the preparation of a cooling composition in the form of a granulate, **characterized in that**
a cooling composition is encapsulated in a spray granulation process, wherein the cooling composition is a composition consisting of
(A) 8 to 10 wt. % cooling active selected from the group consisting of menthone glyceryl acetal (FEMA GRAS 3807), menthone glyceryl ketal (FEMA GRAS 3808), menthol lactate (FEMA GRAS 3748), menthol ethylene glycol carbonate (FEMA GRAS 3805), menthol propylene glycol carbonate (FEMA GRAS 3806), menthol N-ethyloxamate, monomethyl succinate (FEMA GRAS 3810), monomenthyl glutamate (FEMA GRAS 4006), menthoxy-1,2-propanediol (FEMA GRAS 3784), menthoxy-2-methyl-1,2-propanediol (FEMA GRAS 3849) and the menthanecarboxylic acid esters and amides WS-3, WS-4, WS-5, WS-12, WS-14, WS-23 and WS-30, or acyclic carboxamides of the formula (I) 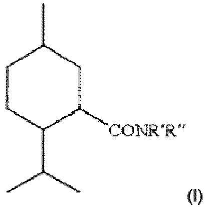 wherein
R' and R" independently of another may be hydrogen, or a hydroxyl group or a branched, unbranched, cyclic alkyl radical having up to 25 carbon atoms, an aryl group having up to 10 carbon atoms selected from substituted and unsubstituted phenyl, phenylalkyl, naphthyl and pyridyl radicals, or mixtures thereof, and
(B) at least 15 % by weight alcohol selected from the group consisting of a C1 to C3 alcohol or mixtures thereof, and
(C) at least 60 % by weight hydrophobic compound selected from the group consisting of essential oils, neutral oils, vegetable oils or mixtures thereof,
wherein the cooling agent (A) is completely dissolved in (B) and (C) in an upstream step to form a homogeneous mixture, wherein the temperature is being maintained at 40°C to 100°C for complete dissolution of (A),
and in the further spray granulation process, the temperature is maintained at 35°C to 65°C to prevent recrystallization of (A),
wherein the granule particles of the cooling composition obtained from the spray granulation in the form of a granule have an average particle size of 0.3 mm to 0.9 mm.

2. Method according to claim 1, **characterized in that** the cooling agent (A) is (1R,25,5R)-N-(4-methoxyphenyl)-5-methyl-2-(1-methylethyl)cyclohexane-carboxamide (FEMA 4681).

3. Method according to any one of claims 1 or 2, **characterized in that** the hydrophobic compound (C) is selected from the group consisting of peppermint oil (menthol), carvone, eucalyptus oil, grapefruit, orange oil, lemon oil, turpentine oil, tea tree oil, clove oil, camphor, rose oil, lavender oil or methyl salicylate, sunflower oil, olive oil, safflower oil, sesame oil, almond oil, algae oil, apricot kernel oil or apricot kernel oil (German: Marillenkernol), argan oil, avocado oil, borage oil or borage seed oil, cashew shell oil, rosehip seed oil, hazelnut oil, jojoba oil, cafe bean oil, camellia oil, macadamia oil, almond oil, papaya seed oil, pistachio oil, castor oil, sea buckthorn oil, sea buckthorn kernel oil, walnut oil or neutral oils with a content of 50-65% caprylic acid, 30-45% capric acid and a small amount of caproic acid, lauric acid and myristic acid or mixtures thereof.

## Revendications

1. Procédé de production d'une composition réfrigérante sous forme de granulés, **caractérisé par le fait**
**qu'**une composition réfrigérante est encapsulée dans un processus de granulation par pulvérisation, dans lequel la composition réfrigérante est une composition qui se compose de
(A) 8 à 10 % en poids d'agent de refroidissement choisi dans le groupe constitué par le menthone glycéryl acétal (FEMA GRAS 3807), le menthone glyceryl cétal (FEMA GRAS 3808), le lactate de mentyle (FEMA GRAS 3748), le carbonate de menthol et d'éthylène glycol (FEMA GRAS 3805), le carbonate de menthol et de propylène glycol (FEMA GRAS 3806), le N-éthyloxamate de menthyle, les succinates de monométhyle (FEMA GRAS 3810), les glutamates de monomenthyle (FEMA GRAS 4006), le menthoxy-1,2-propanediol (FEMA GRAS 3784), le menthoxy-2-méthyl-1,2-propanediol (FEMA GRAS 3849) ainsi que les esters et amides d'acide menthane-carboxylique WS-3, WS-4, WS-5, WS-12, WS-14, WS-23 et WS-30 ou les carboxamides acycliques de formule (I) 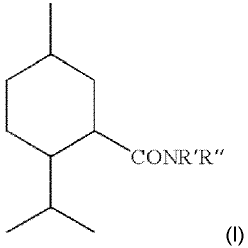 dans laquelle
R' et R" peut être, indépendamment l'un de l'autre, de l'hydrogène, ou un groupe hydroxyle ou un radical alkyle cyclique ramifié, non ramifié comprenant jusqu'à 25 atomes de carbone, un groupe aryle comprenant jusqu'à 10 atomes de carbone, choisi parmi des radicaux phényle, phénylalkyle, naphtyle et pyridyle, substitués et non substitués, ou leurs mélanges, et
(B) au moins 15 % en poids d'alcool choisi dans le groupe constitué par un alcool en C1 à C3 ou des mélanges de ceux-ci, et
(C) au moins 60 % en poids de composé hydrophobe choisi dans le groupe constitué par les huiles essentielles, les huiles neutres, les huiles végétales ou leurs mélanges,
dans lequel l'agent de refroidissement (A) est complètement dissous dans (B) et (C) dans une étape précédente de sorte qu'un mélange homogène est formé, la température étant maintenue entre 40 °C et 100 °C pour dissoudre complètement (A),
et, dans l'autre processus de traitement de granulation par pulvérisation, la température est maintenue entre 35 °C et 65 °C afin d'empêcher une recristallisation de (A),
dans lequel les particules de granulés de la composition de refroidissement sous la forme de granulés, qui sont obtenues à partir de la granulation par pulvérisation présentent une granulométrie moyenne comprise entre 0,3 mm et 0,9 mm.

2. Procédé selon la revendication 1, **caractérisé par le fait que** l'agent de refroidissement (A) est le (1)R,25,5R)-N-(4-méthoxyphényl)-5-méthyl-2-(1-méthylethyl- cyclohexane-carboxamide (FEMA 4681).

3. Procédé selon l'une quelconque des revendications 1 ou 2, **caractérisé par le fait que** le composé hydrophobe (C) est choisi dans le groupe constitué par l'huile de menthe poivrée (menthol), la carvone, l'huile d'eucalyptus, le pamplemousse, l'huile d'orange, l'huile de citron, l'essence de thérébentine, l'huile essentielle d'arbre à thé et de girofle, le camphre, l'huile de rose, l'huile de lavande ou le salicylate de méthyle, l'huile de tournesol, l'huile d'olive, l'huile de carthame, l'huile de sésame et d'amande, l'huile d'algue, l'huile de noyau d'abricot, l'huile d'argan, l'huile d'avocat, l'huile de bourrache ou l'huile de graines de bourrache, l'huile de coquille de cajou, l'huile de graines de cynorhodon, l'huile de noisetier, l'huile de jojoba, l'huile de grains de café, l'huile de camélia, l'huile de Macadamia, l'huile d'amande, huile de graines de papaye, l'huile de pistache, l'huile de ricin, l'huile d'argousier, l'huile de noyaux d'argousier, l'huile de noix ou les huiles neutres ayant une proportion de 50 à 65 % d'acide caprylique, de 30 à 45 % d'acide caprique et une petite proportion d'acide caproïque, d'acide laurique et d'acide myristique ou leurs mélanges.
